# EUROPEAN PATENT SPECIFICATION

(11) **EP 2 958 600 B1**
(45) Date of publication and mention of the grant of the patent: **18.11.2020**
(21) Application number: 14753906.8
(22) Date of filing: 19.02.2014
(51) Int. Cl.: A61L 15/46, A61L 26/00, A61L 15/24, A61L 15/60

(54) **ANTIMICROBIAL HYDROGEL POLYMERS**
ANTIMIKROBIELLE HYDROGELPOLYMERE
POLYMÈRES D'HYDROGEL ANTIMICROBIENS

(30) Priority: 20.02.2013 US 201313772141
(43) Date of publication of application: 30.12.2015
(73) Proprietor: CareFusion 2200 Inc., San Diego, CA 92130 (US); First Water Limited, Marlborough Wiltshire SN8 2RB (GB)
(72) Inventor: TUFTS, Scott A., El Paso, Texas 79912 (US); BALTEZOR, Michael J., Lee's Summit, Missouri 64064 (US); ORTIZ, Moises, San Diego, California 92130 (US); FLORES, Jesus, El Paso, Texas 79932 (US); DEVENS, Jennifer Raeder, San Diego, California 92130 (US); MUNRO, Hugh Semple, Chipping Campden Warwickshire GL55 6QT (GB); BOOTE, Nicholas, Faringdon Oxfordshire SN7 8EZ (GB)
(74) Representative: Banchetti, Marina
(86) International application number: PCT/US2014/017187
(87) International publication number: WO 2014/130567

(56) References cited:
- EP-A2- 2 311 504
- WO-A1-02/38097
- WO-A1-03/051408
- WO-A1-03/051408
- US-A1- 2012 009 275
- US-A1- 2012 328 682
- US-B1- 6 592 912
- EFE ET AL.: 'Synthesis of 4-Acryloylmorpholine-based Hydrogels and Investigation of their Drug Release Behaviors.' J. BRAZ. CHEM. SOC. vol. 24, no. 5, 2013, pages 814 - 820, XP055273649

## Description

### Background of the Invention

### 1. Field of the Invention

The present invention relates generally to antimicrobial hydrogel polymers, and to dressings comprising one or more hydrogel-forming hydrophilic polymers, where the dressings further comprise one or more antimicrobial agents that are released from the hydrogel, preferably in a controlled manner. The dressings may be used for medical, veterinary, and/or cosmetic applications. The dressings are particularly useful in medical applications, and may be applied, for example, as wound dressings. surgical dressings, and percutaneous device insertion site lubricants and dressings. In certain aspects of the invention, the dressings may be beneficially used in methods of accelerating wound healing, preventing and/or reducing the incidence of wound infection, and reducing scarring associated with wounds.

### 2. Description of Related Art

Nosocomial infections are infections which are a result of treatment in a hospital or other healthcare setting, but secondary to the patient's original condition. Infections are considered nosocomial if they first appear 48 hours or more after hospital admission or within 30 days after discharge. This type of infection is also known as a hospital-acquired infection.

About 1.7 million patients get sick in hospitals each year from infections they acquire while in the hospital. Of those 1.7 million, approximately 100,000 die as a result of their infection. (Klevins et al., "Estimating Health Care-Associated Infections and Deaths in U.S. Hospitals, 2002," Public Health Reports Vol. 122 (March/April 2007)) Nosocomial infections may include surgical site infections, urinary tract infections, respiratory infections, vascular catheter-related infections, and septicemia. According to the U.S. Centers for Disease Control (CDC), the most common types of healthcare-acquired infections are urinary tract infections (32 percent), surgical site infections (22 percent), pneumonias (15 percent), and bloodstream infections (14 percent). (See "Questions and Answers about Healthcare-Associated Infections" http://www.cdc.gov/ncidod/dhqp/ hai_qa.html (May 30, 2007).)

Etiologies involved in bloodstream infections commonly include coagulase-negative *Staphylococci*, *Enterococci*, fungi, *Staphylococcus aureus,* Enterobacter species. *Enterococcus faecalis, Staphylococcus epidermidis, Streptococcus viridans, Escherichia coli, Klebsiella pneumoniae, Proteus mirabllis, Pseudomonas aeruginosa,* and *Acinetobacter baumannii* with substantial antimicrobial resistance. These and other microorganisms commonly attach to living and nonliving surfaces, including those of indwelling medical devices.

In order to combat nosocomial infections, the CDC is promoting a campaign based on preventing infections, diagnosing and treating infections appropriately, using antibiotics wisely, and preventing transmission of microbes. Specific recommendations related to the prevention of infection transmission include having health care providers and personnel keep their hands clean at all times, and removing catheters as soon as possible.

However, in many hospitals there is poor adherence to hand hygiene guidelines. Health care workers face many obstacles in their attempt to keep clean hands. They might not be able to find a sink, they have limited time between patients, and after washing their hands as many as 30 times during a work shift they may have serious problems of skin irritation and dryness. Another area for improvement in deterring infection is training in the proper use of medical devices. Medical devices can be portals leading to contamination if not used or inserted properly, or if the health care worker hasn't practiced good hand hygiene. Various medical devices have been associated with hospital-acquired infections and/or biofilm formation, including urinary and venous catheters.

Intravascular catheters are frequently used in modem-day medical practice, particularly in intensive care units (ICUs), and provide necessary vascular access. However, their use puts patients at risk for complications, including local site infection, intravascular catheter-related bloodstream infections (CRBSI), septic thrombophlebitis, endocarditis, and other metastatic infections (e.g., lung abscess, brain abscess, osteomyelitis, and endophthalmitis).

Health-care practitioners insert millions of intravascular catheters each year. The incidence of CRBSI varies by type of catheter, frequency of catheter manipulation, and patient-related factors (e.g., underlying disease and seriousness of illness). Peripheral venous catheters are the devices most frequently used for vascular access. Although the incidence of local or bloodstream infections (BSIs) associated with peripheral venous catheters is usually low, serious infectious complications produce considerable annual morbidity because of the frequency with which such catheters are used. Certain catheters (e.g., pulmonary artery catheters and peripheral arterial catheters) can be accessed multiple times per day for hemodynamic measurements or to obtain samples for laboratory analysis, increasing the potential for contamination and infection. The majority of serious catheter-related infections are associated with central venous catheters (CVCs), especially those that are placed in patients in ICUs. In the ICU setting, the incidence of infection is often higher than in the in-patient or ambulatory setting. In the ICU, central venous access might be needed for extended periods of time. Patients can be colonized with hospital-acquired organisms, and the catheter can be manipulated multiple times per day for the administration of fluids, drugs, and blood products. (O'Grady et al., "Guidelines for the Prevention of Intravenous Catheter-Related Infections," MMWR 51:1-26 (2002).)

Dressings, including surgical dressings, wound dressings, and patches for use with catheters or other percutaneous devices, such as bone fixation pins, are commonly used in the medical field. Although the primary concern with wounds and transcutaneous access sites into the body is the prevention of infection, there are also secondary concerns regarding promoting and accelerating wound healing, and minimizing irritation and scarring at the wound site. Given the attention devoted to prevention of hospital-acquired infections, and the significant increase in the use of long-term catheterization of patients in a wide array of settings, such as hospitals, nursing homes, and home health care, there is a need for dressings and methods for preventing microbial intrusion into wounds and medical device insertion sites.

Hydrogels are hydrophilic polymer networks that are able to swell and retain large amounts of water or other aqueous fluids while maintaining three-dimensional swollen structures. This property anses from the presence of physical and/or chemical crosslinks that prevent dissolution in an aqueous fluid. Hydrogels may be used to produce medical dressings that do not adhere to wounds, and provide moist environments for wound healing. Hydrogel dressings have been used in the treatment of burns, ulcers, and surgical wounds.

U.S. Patent No. 4,931,282 discloses a pressure-sensitive medical sealant composition formed from a crosslinked, swellable polymer matrix of an N-vinyl lactam monomer and a multiethylenically unsaturated compound, a plasticizer, and an antimicrobial, such as iodine, parachlorometaxylenol, or chlorhexidine gluconate. The composition may be used as a wound or burn dressing, or as a sealant to seal junctions between medical instruments penetrating the skin.

U.S. Patent No. 6,683,120 discloses bioadhesive compositions for use as skin adhesives. The compositions are formed by polymerizing an aqueous reaction mixture containing water, at least one monomer that forms a hydrogel upon polymerization, and optionally at least one crosslinking agent. The compositions may also be used to prepare bandages and wound dressings. An antimicrobial agent such as citric acid or stannous chloride may also be included in the hydrogel.

U.S. Published Application No. 2007/0282237 discloses absorbent materials that are flexible, skin-conformable, moisture-absorbent sheets having a hydrogel associated therewith. Absorbent articles comprising the absorbent material are also provided, such as dressings. The hydrogel may include antimicrobial agents.

U.S. Published Application No. 2009/0187130 discloses adhesive hydrogel dressings that include an adhesive hydrogel pad, a backing layer, and an adhesive layer on the backing layer that faces the hydrogel pad. The adhesive layer and backing form a perimeter around the hydrogel to hold the pad in place. The hydrogel may comprise a swellable, crosslinked poly(N-vinyl lactam), a swelling agent, and a modifying polymer in an amount sufficient to form a pressure-sensitive adhesive. An antimicrobial agent such as chlorhexidine gluconate may be included in the hydrogel composition.

WO 03/051408 A1 discloses a hydrogel composition comprising a plasticized cross-linked hydrophilic polymer of acryloylmorpholine, optionally with one or more copolymers, to be used for wound and burn dressings, biomedical electrodes and other applications where skin compatibility is required. The hydrogel is formed by polymerization of an acryloylmorpholine monomer, with glycerol and water, as well as one o more free-radical photoinitiators.

There is a need in the art for dressings, particularly hydrogel-based dressings comprising one or more hydrophilic polymers,that preferably provide controlled release of one or more antimicrobial agents. The dressings may be used for medical, veterinary, and/or cosmetic applications. The dressings are particularly useful in medical applications, and may be applied, for example, as wound dressings, surgical dressings, and catheter site dressings. The dressings may beneficially be used to accelerate wound healing, prevent and/or reduce the incidence of wound infection, and reduce scarring associated with wound healing.

### Summary of the Invention

It has been discovered that certain antimicrobial dressings, especially those formed from hydrogels, may suffer from problems associated with the rate of release of the antimicrobial agent from the dressing. The antimicrobial agent may be bound too tightly by the composition used to form the dressing, resulting in reduced effectiveness of the dressing in controlling growth of microbes and preventing wound infection. Alternatively, the antimicrobial agent may be released too rapidly, resulting in the patient being exposed to high levels of a potentially toxic substance, and causing failure of the dressing to provide long-term antimicrobial protection, thereby necessitating more frequent dressing changes. These issues can be particularly problematic when the antimicrobial dressing is being used in the care of percutaneous access devices such as catheters.

The present invention meets the unmet needs of the art, as well as others. by providing antimicrobial hydrogel dressings in which the hydrogel component preferably provides controlled release of the antimicrobial agent to the wound site. The antimicrobial hydrogel dressings of the invention are particularly beneficial for use in maintaining percutaneous access sites, and preventing infections.

The invention relates to antimicrobial hydrogels and wound dressings formed using such antimicrobial hydrogels, as defined by the claims.

One or more organic plasticizers are present in the antimicrobial hydrogel, where the organic plasticizer(s) is/are polyhydric alcohols (e.g., glycerol). Glycerol is a presently-preferred plasticizer. Another presently-preferred plasticizer is the ester derived from boric acid and glycerol. When present, the organic plasticizer may be provided in an amount of up to about 45% by weight of the polymerization reaction mixture.

In certain aspects, the non-ionic based polymer is a polymer comprising 4-acryloylmorpholine monomers. The antimicrobial agent may be a biguanide antimicrobial agent. The antimicrobial agent is preferably selected from the group consisting of octenidine dihydrochloride, olanexidine, alexidine, chlorhexidine digluconate. chlorhexidine diacetate, chlorhexidine dihydrochloride, chlorhexidine diphosphanilate, alexidine, olanexidine, and polyhexamethylene biguanide. In further aspects, the hydrogel does not significantly swell as the antimicrobial agent is released.

An additional aspect of the invention relates to wound dressings formed using the antimicrobial hydrogels of the invention. The wound dressings preferably provide controlled release of the antimicrobial agent from the dressing. The dressing may be selected from the group consisting of a wound dressing, a surgical dressing, and a catheter site dressing.

Further aspects of the invention relate to methods of accelerating healing of wounds, and/or preventing or reducing the incidence of wound infection. These methods include applying the wound dressing of the present invention to a wound.

Other novel features and advantages of the present invention will become apparent to those skilled in the art upon examination of the following or upon learning by practice of the invention.

### Brief Description of the Drawings

For a more complete understanding of the present invention, the needs satisfied thereby, and the objects, features, and advantages thereof, reference now is made to the following description taken in connection with the accompanying drawings.
Figure 1 is a graph depicting the amount of chlorhexidine gluconate (CHG) remaining on tested patches vs. duration of patch wear (in days) for each test article.
Figure 2 is a box plot depicting the amount of CHG released by the tested patches over the 7-day test period.
Figure 3 is a scatter plot comparing the amount of CHG released into a liquid extraction medium over a three-hour period.

### Detailed Description of the Preferred Embodiments

The present invention relates to compositions for providing antimicrobial agents onto body surfaces of mammals, including humans. The compositions preferably provide the active agents in a controllable manner.

The hydrogels and wound dressings of the present invention are as defined by the claims and provide controlled release of one or more antimicrobial agents. The controlled release may be in the form of a burst release. Following release of the antimicrobial agents, the hydrogel may retain a sufficient amount of the antimicrobial agents therein to reduce and/or eliminate the growth of microbes that contact the dressing. The hydrogel dressings also preferably permit the antimicrobial agents to be released in a manner that prevents growth of microbes in and around wound sites, and reduces the number of dressing changes required to maintain antimicrobial effectiveness.

### Antimicrobial Hydrogels

The antimicrobial polymers and dressings containing them are formed using hydrogel polymers, which for purposes of the present invention encompass hydrophilic polymers that are capable of absorbing water or other aqueous fluids (e.g., wound exudates). Although cationic, anionic or non-ionic hydrogels may be used in accordance with the invention, non-ionic, hydrophilic hydrogels are preferred, especially when the antimicrobial agent is a biguanide, such as octenidine dihydrochloride, chlorhexidine digluconate, alexidine, olanexidine, and polyhexamethylene biguanide.

Hydrogels may be formed using a variety of hydrophilic monomers, including, but not limited to, hydroxyl functional monomers (e.g., diallyl maleate, hydroxypolyethoxy allyl ether, hydroxyethyl acrylate, hydroxyethyl methacrylate, polyethoxyethyl methacrylate, N,N-dimethylacrylamide, ethylene glycol dimethacrylate), acid functional monomers (e.g., acrylic acid and derivatives, methacrylic acid and derivatives, beta-carboxyethyl acrylate, vinylbenzoic acid), and sulfonated monomers (e.g., allyl ether suffonate, 2-propene-1-sulfonic acid). Other monomers may include 2-cyanoethyl acrylate, and allyl phenyl ether. Preferred hydrophilic monomers for use in the hydrogels of the invention are acrylates, methacrylates, and acrylamides, and derivatives thereof. The polymers of the invention may be formed using a single type of monomer, or a blend of two, three, or more different monomers, in order to achieve the desired functional characteristics. The polymers may be formed by providing an aqueous solution comprising a monomer and/or comonomers, an optional crosslinker, and an initiator. The monomer and/or comonomers are then polymerized in the solution. At least a portion of the aqueous solution is retained as part of the hydrogel.

In a preferred embodiment, in preparing hydrogel compositions in accordance with the invention, the ingredients are be mixed to provide a reaction mixture in the form of an initial pre-gel aqueous-based liquid formulation, which is then converted into a hydrogel by a free radical polymerization reaction. The free radical polymerization reaction may be a photopolymerization reaction. Photopolymerization may be achieved using photoinitiators, optionally together with one or more other initiators, such as heat and/or ionizing radiation. Photoinitiation may be accomplished by subjecting the pre-gel reaction mixture containing an photoinitiation agent to ultraviolet (UV) light after it has been spread or coated as a layer on siliconized release paper or other solid substrate. The incident UV intensity, at a wavelength in the range from 240 to 420nm. is typically greater than about 10mW/cm². The processing will generally be carried out in a controlled manner involving a precise predetermined sequence of mixing and thermal treatment or history. The UV irradiation time scale should ideally be less than 60 seconds, and preferably less than 10 seconds to form a gel with better than 70% conversion of the monomers, preferably better than 80% conversion of the monomers. Those skilled in the art will appreciate that the extent of irradiation will be dependent on a number of factors, including the UV intensity, the type of UV source used, the photoinitiator quantum yield, the amount of monomer present, the nature of the monomers) present, the presence of dissolved oxygen, the presence of polymerization inhibitor, the thickness of the reaction mixture when coated onto the substrate, and the nature of substrate onto which the reaction mixture is coated.

Hydrogels for use in the dressings of the invention are preferably selected from those described in U.S. Patent Nos. 6,683,120 and 6,896,903. A particularly preferred non-ionic hydrogel polymer is formed by combining the monomer 4-acryloylmorpholine with glycerol, and reacting these components in water in the presence of a crosslinking agent. However, it is understood that other hydrogel polymers may also be used in accordance with the invention.

One or more antimicrobial agents that are chemically compatible with the hydrogel may be incorporated into or onto the hydrogel. In some aspects of the invention, the antimicrobial agent is incorporated via adsorption and/or absorption. The antimicrobial agent may be incorporated in an amount that is sufficient to slow the growth rate of microbes that come into contact with the hydrogel or hydrogel dressing, preferably the antimicrobial agent is provided in an amount that is sufficient to arrest the growth rate of microbes that come into contact with the hydrogel or hydrogel dressing, and more preferably the antimicrobial agent is provided in an amount that is sufficient to kill microbes that come into contact with the hydrogel or hydrogel dressing. According to some aspects, the antimicrobial agent is provided in an amount that is safe for use on skin. According to further aspects, the antimicrobial agent is used in an amount that is safe for use on open wounds, including percutaneous access sites. It will be appreciated that the amounts required to accomplish these goals will vary based on the specific antimicrobial agent being used.

Generally speaking, the antimicrobial agent may be provided in an amount that is necessary to achieve the desired effect, which will vary based on factors including, but not limited to, the microorganisms that are likely to be encountered during use of the article, the manner in which the antimicrobial agent is incorporated into the article (i.e., as a coating, or embedded within the article), the specific antimicrobial agents and hydrogel polymer selected, and the particular application in which the antimicrobial hydrogel is used.

Preferably the antimicrobial agent is included in or on the hydrogel in amounts that are adequate to kill or restrict the growth of one or more of the following microbes: coagulase-negative *Staphylococci, Enterococci,* fungi, *Candida albicans, Staphylococcus aureus, Enterobacter* species, *Enterococcus faecalis, Staphylococcus epidermidis, Streptococcus viridans, Escherichia coli, Klebsiella pneumoniae, Proteus mirabilis, Pseudomonas aeruginosa, Acinetobacter baumannii, Burkholderia cepacia,* Varicella. *Clostridium difficile, Clostridium serdellii,* Hepatitis A, Hepatitis B, Hepatitis C, HIV/AIDS, methicillin-resistant *Staphylococcus aureus* (MRSA), mumps, norovirus, parvovirus, poliovirus, rubella. SARS, S. *pneumoniae* (including drug resistant forms), vancomycin-intermediate *Staphylococcus aureus* (VISA), vancomycin-resistant *Staphylococcus aureus* (VRSA), and vancomycin-resistant *Enterococci* (VRE). It is considered to be within the ability of one skilled in the art to determine such amounts.

Any antimicrobial agent capable of associating with the hydrogel polymers of the invention, while still retaining ability to kill or inhibit the growth of bacteria, fungi, viruses and/or parasites, may be used in accordance with the present invention. For example, suitable antimicrobial agents include, without limitation, biguanides (e.g., chlorhexidine, chlorhexidine salts (such as chlorhexidine digluconate, chlorhexidine diacetate, chlorhexidine dihydrochloride, and chlorhexidine diphosphanilate), polyhexamethylene biguanide (PHMB), alexidine, alexidine salts, olanexidine, olanexidine salts); pyridines (e.g., benzalkonium chloride, tridodecyl methyl ammonium chloride, didecyl dimethyl ammonium chloride, chloroallyl hexaminium chloride, benzethonium chloride, methylbenzethonium chloride, cetyl trimethyl ammonium bromide, cetyl pyridinium chloride, dioctyldimethyl ammonium chloride); quaternary ammonium compounds (including monoalkyltrimethyl ammonium salts, monoalkyldimethylbenzyl ammonium salts, dialkyldimethyl ammonium salts, heteroaromatic ammonium salts, polysubstituted quaternary ammonium salts, bis-quaternary ammonium salts, and polymeric quaternary ammonium salts); heavy metal compounds (e.g., silver chloride, silver oxide, silver sulfadiazine, colloidal silver, stannous chloride, stannous fluoride); bisphenols (e.g., triclosan and hexachlorophene); octenidine and octenidine salts (such as octenidine dihydrochloride); acids (e.g., citric acid, acetic acid, hydrochloric acid, salicylic acid, boric acid, lactic acid, sulfuric acid, and salts of any of these); rifampin; minocycline; iron-sequestering glycoproteins (e.g., lactoferrin, ovotransferrin/conalbumin); cationic polypeptides (e.g., protamine, polylysine, lysozyme); surfactants (e.g., SDS, Tween-80, surfactin, Nonoxynol-9); alkylparabens (including methyl, ethyl, propyl, and butyl parabens); and zinc pyrithione. Further preferred antimicrobial agents include broad-spectrum antibiotics, antifungals, and antiseptic agents. Chemical classes of antibiotics that may be incorporated into the hydrogels of the invention include, but are not limited to, beta-lactams (including penicillins, cephalosporins, and semisynthetic beta-lactams); clavulanic acid; mono-bactams; carboxypenems; aminoglycosides; glycopeptides; lincomycins; macrolides; polypeptides; polyenes; rifamycins; tetracyclines (including semisynthetic tetracycline); chloramphenicol; quinolones (including fluoroquinolones); sulfonamides; and growth factor analogs. Antifungals may include amphotericin B; nystatin; flucytosine; and azoles (including ketaconazole, itraconazole, and fluconazole). Antiseptic agents may include iodine; iodophors (e.g., PVP-iodine); iodine salts (including potassium iodine); methenamine, nitrofurantoin; nalidixic acid thimerosal; and peroxygen compounds such as hydrogen peroxide, organic peroxides (including peracidic acid, performic acid, perpropionic acid), and inorganic peroxides (including perborates, permanganates).

According to preferred aspects, the hydrogel polymer contains an antimicrobial agent that is a cationic antiseptic, preferably a biguanide, more preferably a bipyridine, and most preferably a chlorhexidine salt. According to more preferred aspects, the antimicrobial agent is chlorhexidine digluconate, and it is included in the hydrogel polymer in an amount ranging from 0.1 - 6.0% w/w, preferably from 1.0 - 5.0% w/w, and more preferably from 1.4 to 4.0% w/w.

The antimicrobial agent is provided on the surface of the hydrogel. In accordance with this aspect of the invention, the antimicrobial agent may be applied using any technique generally suitable for topical application of an antimicrobial agent to the formed hydrogel polymer. Such techniques may be based on the carrier in which the antimicrobial agent is contained, and include spraying, dipping, or spreading.

A method for incorporating an antimicrobial agent into the hydrogel is an imbibing technique. One such method includes the step of contacting a hydrogel with an aqueous solution containing an antimicrobial agent, where the antimicrobial agent may be permitted to diffuse into the hydrogel, and the step of contacting the hydrogel with an antimicrobial solution may optionally be repeated as necessary to reach a desired concentration of antimicrobial agent. Another imbibing method includes the steps of dehydrating a hydrogel polymer and then rehydrating it using a solution containing an antimicrobial agent. These techniques may be used to incorporate any water-soluble antimicrobial agent into a hydrogel.

Additional methods that may be used to incorporate the antimicrobial agent into the hydrogel include, without limitation, inkjet printing, soaking in a bath containing the antimicrobial agent either batchwise or continuously, contacting the hydrogel with a transfer film, or applying a product liner to the hydrogel that has been pre-coated with the antimicrobial agent.

The hydrogels used in the present invention are produced using a solution polymerization technique, wherein the polymerization solution forms part of the hydrogel. This technique is used to achieve a heterogeneous structure in the hydrogel such that the surface region of the dressing is less crosslinked than the bulk interior region. The active agent present in the surface region is therefore more easily released than the active agent contained in the bulk. Although not wishing to be bound by theory, it is believed that this structure, coupled with dispersing the antimicrobial agent into the hydrogel through the surface (e.g.. by imbibing or other application technique) result in the surface region of the hydrogel incorporating the antimicrobial agent at a higher concentration than the bulk interior region. This produces an antimicrobial hydrogel that surprisingly is able to provide burst release of an antimicrobial agent on contact with skin, without requiring significant swelling of the hydrogel to attain the release. Such hydrogels are also beneficially able to retain a sufficient quantity of the antimicrobial agent within the hydrogel to provide an antimicrobial barrier.

Without wishing to be bound by theory, burst release is believed to be attained when a molecule, e.g., an antimicrobial agent such as chlorhexidine. rapidly diffuses out of a hydrogel. This phenomenon is believed to depend on the level of crosslinking and the molecular weight between cross links (known as the mesh size or pore size) of the hydrogel. if the mesh size of the polymer is small relative to the size of the molecule, then diffusion is very constrained; likewise, if the mesh size is large relative to the size of the molecule then diffusion can readily occur. As a hydrogel swells, its effective mesh size can increase, thereby facilitating the release of the molecule. In a hydrogel having a less crosslinked, more open structure, and including a high concentration of the molecule, the molecule may be released in a manner that is rapid or burst like.

It has been discovered in accordance with the present invention that when a hydrogel is used to provide an active agent, it is desirable that the hydrogel be able to quickly release a quantity of the active agent without requiring a significant degree of swelling. When the active agent is an antimicrobial agent, this rapid initial release or "burst" release of the active agent facilitates antisepsis, and also aids in retaining a sufficient quantity of active agent within the hydrogel to provide an antimicrobial barrier for the length of time that the hydrogel Is in contact with the skin.

According to some aspects of the invention, the burst release occurs when the hydrogel has swelled by 50% or less, 40% or less, 30% or less, 20% or less; preferably the burst release occurs when the hydrogel has swelled by 15% or less, 1 2% or less, 8% or less, 6% or less; and more preferably the burst release occurs when the hydrogel has swelled by 5% or less, 4% or less, 3% or less, 2% or less, or 1 % or less.

According to further aspects of the invention, the burst release results in the release of 75% or less of the antimicrobial agent provided in the dressing, 65% or less, 50% or less; preferably the burst release results in the release of 40% or less, 30% or less; and more preferably the burst release results in the release of 25% or less, 20% or less, 15% or less, or 10% or less of the antimicrobial agent provided in the dressing.

According to further aspects, the antimicrobial hydrogels of the present invention may beneficially provide an initial burst release of an antimicrobial agent, followed by the retention of a level of the antimicrobial agent that is sufficient to impart antimicrobial effectiveness to the hydrogel for an extended period of time. The hydrogel may retain antimicrobial efficacy for the length of time that it is in contact with a patient's skin. For example, the burst release may take place over a period of up to about 48 hours, or about 36 hours; preferably up to about 24 hours; and more preferably over a period of up to about 12 hours, about 8 hours, or about 4 hours. The antimicrobial hydrogel may also retain sufficient antimicrobial agent to remain antimicrobially effective for a period of about 3 days following the initial burst release; preferably about 4 days or 5 days following the initial burst release; and more preferably about 6, 7, or 8 days following the initial burst release.

The retention of the antimicrobial agent within the hydrogel following the initial burst release of antimicrobial agent may be such that none or substantially none (less than 1%, 2%, or 3%) of the antimicrobial agent remaining in the hydrogel is released. The hydrogel may also retain sufficient antimicrobial agent following the initial burst release to render the hydrogel antimicrobially effective, while providing sustained release of antimicrobial agent. Such sustained release may result in less than 25%, 20%, or 15%, preferably less than 10% or 8%, and more preferably less than 6%, 5%, or 4% of the remaining antimicrobial agent being released from the hydrogel following the initial burst release.

### Hydrogel Dressings

Regardless of how the antimicrobial agent has been associated with the hydrogel, dressings may be formed from the hydrogel using standard components and techniques. Various wound dressings, surgical dressings, and bum dressings are envisioned in accordance with the invention. For example, the hydrogel may be incorporated into a wound dressing having one or more of a backing layer, a release liner, an adhesive, and any other components commonly-associated with bum, wound, and catheter dressings. The dressings may also be provided in customized shapes for use on different areas of the body, or for use to protect the area around a vascular access catheter, for example. A particular dressing structure that is envisioned within the scope of the present invention is a catheter patch.

The antimicrobial hydrogel dressings of the present invention are surprisingly able to provide burst release of an antimicrobial agent on contact with skin. without requiring significant swelling of the hydrogel within the dressing to attain the desired release profile. According to some aspects of the invention, the burst release occurs when the hydrogel has swelled by 50% or less, 40% or less, 30% or less, 20% or less; preferably the burst release occurs when the hydrogel has swelled by 1 5% or less, 12% or less, 8% or less, 6% or less; and more preferably the burst release occurs when the hydrogel has swelled by 5% or less, 4% or less, 3% or less, 2% or less, or 1% or less. According to further aspects of the invention, the burst release results in the release of 75% or less of the antimicrobial agent provided in the dressing, 65% or less, 50% or less; preferably the burst release results in the release of 40% or less, 30% or less; and more preferably the burst release results in the release of 25% or less, 20% or less. 15% or less, or 10% or less of the antimicrobial agent provided in the dressing.

According to further aspects, the dressings of the present invention may beneficially provide an initial burst release of an antimicrobial agent, followed by the retention within the dressing of a level of the antimicrobial agent that is sufficient to impart antimicrobial effectiveness to the dressing for an extended period of time. For example, the burst release may take place over a period of up to about 48 hours, or about 36 hours; preferably about 24 hours; and more preferably over a period of from about 12 hours, about 8 hours, or about 4 hours. The antimicrobial hydrogel dressing may retain sufficient antimicrobial agent to remain antimicrobially effective for a period of about 3 days following application of the dressing; preferably about 4 days or 5 days following application of the dressing; and more preferably about 6, 7, or 8 days following application of the dressing.

The dressings of the present invention may retain antimicrobial agent following the initial burst release of antimicrobial agent. The retention may be such that none or substantially none (less than 1%, 2%, or 3%) of the antimicrobial agent remaining in the dressing is released, and is instead available to act as a barrier for the length of time that the dressing is in use. The dressing may also retain sufficient antimicrobial agent following the initial burst release to render the dressing antimicrobially effective, while providing sustained release of antimicrobial agent. Such sustained release may result in less than 25%, 20%, or 15%, preferably less than 10% or 8%, and more preferably less than 7%, 6%, 5%, or 4% of the remaining antimicrobial agent being released from the dressing following the initial burst release.

### Methods

The hydrogels and dressings containing them may be beneficially used to carry out methods for providing an initial burst of an active agent to a body surface of a mammal, preferably a human. The hydrogels and dressings may initially provide a burst release of active agent, but then retain a sufficient quantity of the antimicrobial agent within the hydrogel/dressing to provide an antimicrobial barrier. The antimicrobial hydrogels and dressings containing them may be beneficially used in methods of treating wounds, including surgical wounds, burns, chronic wounds such as bedsores and diabetic ulcers, and wounds created by insertion of medical devices through the skin. The antimicrobial hydrogels and dressings containing them are particularly beneficial when used to protect catheter access sites.

The antimicrobial hydrogels and dressings of the present invention may be used in accordance with methods of accelerating healing of a wound, by applying the antimicrobial hydrogel or dressing to a wound site. According to some aspects, the acceleration in healing is evaluated as compared to antimicrobial hydrogels and dressings not providing an initial burst of active agent upon application to a body surface. The acceleration in healing of infections may be at least about 10%, preferably at least about 15%, and more preferably at least about 20%.

The antimicrobial hydrogels and dressings of the present invention may be used in accordance with methods of preventing and/or reducing the incidence of wound infections, by applying the antimicrobial hydrogel or dressing to a wound site. According to some aspects, the incidence of infection is reduced as compared to antimicrobial hydrogels and dressings not providing an initial burst of active agent upon application to a body surface. The reduction in the incidence of infection may be at least about 10%, preferably at least about 15%, and more preferably at least about 20%.

### EXAMPLES

### Example 1

### Step A

A hydrogel was formed by adding 35.7 g of 4-acryloylmorpholine (CAS No. 5117-12-4) to a previously-prepared aqueous mixture including 35.7 g glycerol (CAS No. 56-81-5) and 28.6 g water. The mixture was stirred for 30 minutes. A solution of crosslinker and photoinitiator was prepared by adding 0.191 g of IRR280 (PEG400 diacrylate, UCB Chemicals) to 0.0191 g of Darocur 4265 (Ciba Specialty Chemicals). This was added to the mixture comprising 4-acryloylmorpholine and stirred for one hour while covered to exclude light. 50 g of the mixture was coated on a tray lined with polyurethane film (Inspire 2301, Exopack, UK) at a coat weight of 1.4 kg/m². The mixture was cured in the laboratory by passing the tray under a medium pressure mercury vapor ultraviolet (UV) emitting light operating at a power level of 80 W/cm, where the tray was passed under the light three times at a speed of 7 m/min. The cured gel was covered with a siliconized high density polyethylene (HDPE) top liner.

### Step B

The gel formed in Step A was then packaged in a heat-sealed polyfoil pouch (PP0038J, Protective Packaging, UK) and exposed to 25-40 kGy gamma radiation (Isotron, UK). This step was undertaken in order to remove residual monomer.

### Step C

After removal of the siliconized high-density polyethylene liner from the hydrogel an aqueous solution of chlorhexidine gluconate (20% w/v, Medichem, Spain) was spread across the surface and absorbed by the hydrogel such that approximately 0.155 kg/m² of chlorhexidine gluconate solution was provided within the gel.

### Step D

Once the hydrogel was formed and associated with the antimicrobial agent (Step C), it was used to prepare catheter patches. Catheter patches having an area of 10.45 cm² were prepared.

### Example 2

Hydrogel catheter patches prepared in accordance with Example 1 were tested to determine the amount of antimicrobial agent that is released from the catheter patch over time following application to healthy skin, and compared with existing catheter patch products,

One comparative product is the Ethicon BIOPATCH® Protective Disk with CHG (product code 4151), which is a 1.9 cm (¾ inch) disk having a 1.5 mm center hole and a radial slit. The product label indicates that the amount of CHG provided in the product is 52.5 mg. The patch is formed from a urethane foam.

Another comparative product is the 3M Tegaderm CHG IV Securement Dressing (catalog number 1660), which is a 2.75 x 3.375 inch dressing. The product label indicates that the amount of CHG provided in the product is 15 mg. The patch is formed from a hydrogel.

The testing protocol was as follows:

Seven of each of the three different types of patches were applied to 13-15 healthy adult study subjects for a period 7 days. All 7 patches were applied on day 0, and one patch was removed from the subject each day for testing. Therefore, on day 3, the subject had 4 remaining patches, etc. Additionally, the location of the removed patches for each day was randomized among the patients. Patches that were not applied to human subjects were used as controls, and these results are shown for day 0.

CHG was extracted from the patches by placing the dressing (with any release liners or backing layers removed) in a bottle containing 100 mL of a 75% methanol extraction solution. The bottle containing the dressing and extraction solution was agitated for at least 6 hours at 180 rpm using a platform shaker, at room temperature. The extraction solution was then transferred to a sample vial and diluted by a factor of 100.

The extraction solution sample was then subjected to HPLC analysis to identify and quantify the compounds released from the dressings. Agilent Technologies 1100 and 1200 series HPLC apparatus were used to conduct the testing, and were standardized using a 0.3131 mg/mL solution of chlorhexidine gluconate in 25% methanol solution acidified by dropwise addition of glacial acetic acid. The mobile phase composition was adjusted so that the retention time for chlorhexidine gluconate was from 9 to 11 minutes. Amounts of chlorhexidine gluconate released from the dressings were determined based on the areas of the peaks, and the retention time of the peaks was used to determine that the component corresponded to chlorhexidine gluconate. Results were averaged for the patches tested, and standard deviations were calculated based on these results.

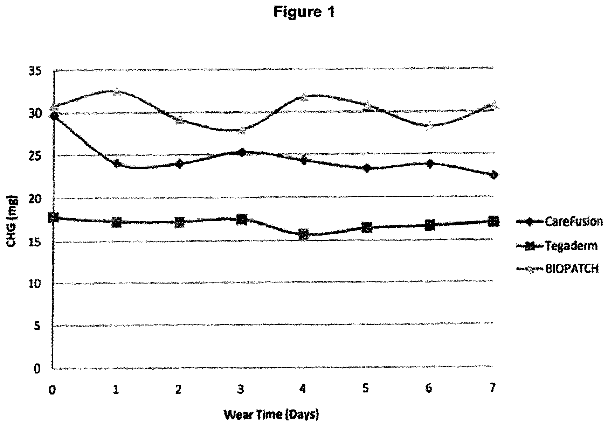

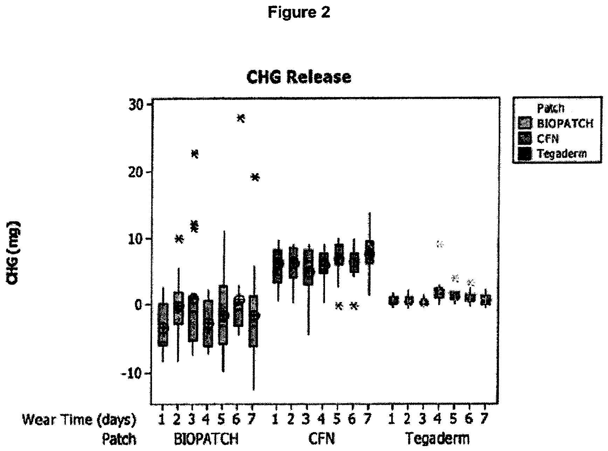

Figures 1 and 2 shows the mean/standard deviation for the assays to determine the total amount of CHG remaining in each patch following application, as well as the amount of CHG released per test article, over the 7-day test period.

### Example 3

Hydrogel catheter patches prepared in accordance with Example 1 were tested to determine the release profile of an antimicrobial agent that is provided in a catheter patch, where the catheter patch exhibited swelling due to absorption of solvent. The antimicrobial agent was extracted using a water/methanol solvent over a period of about three hours, and the release was monitored by UV absorption.

The inventive products were compared with the 3M Tegaderm CHG IV Securement Dressing (catalog number 1660) used in Example 2.

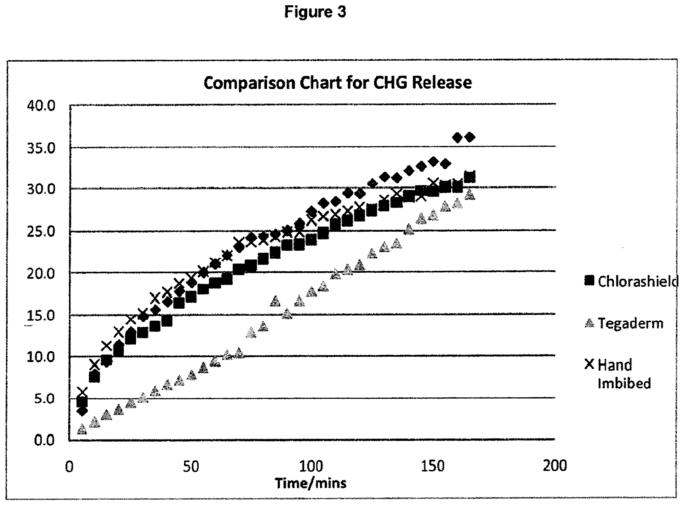

Results of the extraction are shown in Figure 3, and demonstrate that an initial burst of antimicrobial agent is released from the inventive products, whereas the comparative Tegaderm product did not exhibit initial burst release of the antimicrobial agent.

## Claims

1. An antimicrobial hydrogel formed by reacting an acryloylmorpholine monomer and a polyhydric alcohol in a solution polymerization to form a hydrogel wherein the polymerization solution forms part of the hydrogel, and dispersing an antimicrobial agent through a surface of the hydrogel to form the antimicrobial hydrogel,
wherein the antimicrobial concentration in a surface region of the antimicrobial hydrogel is larger than the antimicrobial concentration in a bulk interior of the antimicrobial hydrogel, and wherein from about 10 to about 50% of the antimicrobial agent is released from the hydrogel over a period of less than 24 hours.

2. The antimicrobial hydrogel of claim 1, wherein following the release of about 10 to about 50% of the antimicrobial agent over a period of less than 24 hours, the remaining portion of the antimicrobial agent is substantially retained in the hydrogel.

3. The antimicrobial hydrogel of claim 1, wherein the hydrogel comprises polymers of 4-acryloylmorpholine monomers and glycerol.

4. The antimicrobial hydrogel of claim 1, wherein the antimicrobial agent is selected from the group consisting of bis-biguanide salts (chlorhexidine digluconate, chlorhexidine diacetate, chlorhexidine dihydrochloride, chlorhexidine diphosphanilate), rifampin, minocycline, silver compounds (silver chloride, silver oxide, silver sulfadiazine), triclosan, octenidine dihydrochloride, olanexidine, alexidine, quaternary ammonium compounds (benzalkonium chloride, tridodecyl methyl ammonium chloride, didecyl dimethyl ammonium chloride, chloroallyl hexaminium chloride, benzethonium chloride, methylbenzethonium chloride, cetyl trimethyl ammonium bromide, cetyl pyridinium chloride, dioctyldimethyl ammonium chloride), iron-sequestering glycoproteins (lactoferrin, ovotransferrin/conalbumin), cationic polypeptides (protamine, polylysine, lysozyme), surfactants (SDS, Tween-80, surfactin, Nonoxynol-9), zinc pyrithione, broad-spectrum antibiotics (quinolones, fluoroquinolones, aminoglycosides and sulfonamides), and antiseptic agents (iodine, methenamine, nitrofurantoin, nalidixic acid), and combinations thereof.

5. A wound dressing formed using the antimicrobial hydrogel of claim 1, wherein the antimicrobial hydrogel does not significantly swell as the antimicrobial agent is released.

6. The dressing of claim 5, wherein the dressing is selected from the group consisting of a wound dressing, a surgical dressing, and a catheter site dressing.

7. The wound dressing according to claim 5 for use in accelerating healing of a wound to which said wound dressing is applied.

8. The wound dressing according to claim 5 for use in preventing and/or reducing the incidence of infection in a wound to which said wound dressing is applied.

## Patentansprüche

1. Antimikrobielles Hydrogel, gebildet durch Umsetzen eines Acryloylmorpholinmonomers und eines polyhydrischen Alkohols in einer Lösungspolymerisation, um ein Hydrogel zu bilden, wobei die Polymerisationslösung Teil des Hydrogels bildet, und Dispergieren eines antimikrobiellen Wirkstoffs durch eine Oberfläche des Hydrogels hindurch, um das antimikrobielle Hydrogel zu bilden,
wobei die Konzentration des antimikrobiellen Mittels in einem Oberflächenbereich des antimikrobiellen Hydrogels größer ist als die Konzentration des antimikrobiellen Mittel in einem inneren Hauptteil des antimikrobiellen Hydrogels, und wobei von etwa 10 bis etwa 50 % des antimikrobiellen Wirkstoffs über einen Zeitraum von weniger als 24 Stunden aus dem Hydrogel freigesetzt wird.

2. Antimikrobielles Hydrogel nach Anspruch 1, wobei der Freisetzung von etwa 10 bis etwa 50 % des antimikrobiellen Wirkstoffs über einen Zeitraum von weniger als 24 Stunden folgend der verbleibende Anteil des antimikrobiellen Wirkstoffs im Wesentlichen in dem Hydrogel zurückgehalten wird.

3. Antimikrobielles Hydrogel nach Anspruch 1, wobei das Hydrogel Polymere von 4-Acryloylmorpholinmonomeren und Glycerol umfasst.

4. Antimikrobielles Hydrogel nach Anspruch 1, wobei der antimikrobielle Wirkstoff aus der Gruppe, bestehend aus Bisbiguanidsalzen (Chlorhexidindigluconat, Chlorhexidindiacetat, Chlorhexidindihydrochlorid, Chlorhexidindiphosphanilat), Rifampin, Minocyclin, Silberverbindungen (Silberchlorid, Silberoxid, Silbersulfadiazin), Triclosan, Octenidindihydrochlorid, Olanexidin, Alexidin, quartären Ammoniumverbindungen (Benzalkoniumchlorid, Tridodecylmethylammoniumchlorid, Didecyldimethylammoniumchlorid, Chlorallylhexaminiumchlorid, Benzethoniumchlorid, Methylbenzethoniumchlorid, Cetyltrimethylammoniumbromid, Cetylpyridiniumchlorid, Dioctyldimethylammoniumchlorid), eisensequestrierenden Glycoproteinen (Lactoferrin, Ovotransferrin/Conalbumin), kationischen Polypeptiden (Protamin, Polylysin, Lysozym), Tensiden (SDS, Tween-80, Surfactin, Nonoxynol-9), Zinkpyrithion, Breitband-Antibiotika (Chinolone, Fluorchinolone, Aminoglycoside und Sulfonamide), und antiseptischen Wirkstoffen (Iod, Methenamin, Nitrofurantoin, Nalidixinsäure) und Kombinationen davon ausgewählt ist.

5. Wundverband, gebildet unter Verwendung des antimikrobiellen Hydrogels nach Anspruch 1, wobei das antimikrobielle Hydrogel nicht signifikant anschwillt, wenn der antimikrobielle Wirkstoff freigesetzt wird.

6. Verband nach Anspruch 5, wobei der Verband ausgewählt ist aus der Gruppe, bestehend aus einem Wundverband, einem chirurgischen Verband und einem Einstichstellenverband.

7. Wundverband nach Anspruch 5 zur Verwendung beim Beschleunigen des Heilens einer Wunde, auf die der Wundverband aufgetragen wird.

8. Wundverband nach Anspruch 5 zur Verwendung beim Verhindern und/oder Verringern des Auftretens von Infektion in einer Wunde, auf die der Wundverband aufgetragen wird.

## Revendications

1. Hydrogel antimicrobien formé par mise en réaction d'un monomère d'acryloylmorpholine et d'un alcool polyhydrique dans une polymérisation en solution pour former un hydrogel dans lequel la solution de polymérisation fait partie de l'hydrogel, et par dispersion d'un agent antimicrobien à travers une surface de l'hydrogel pour former l'hydrogel antimicrobien,
dans lequel la concentration antimicrobienne dans une région de surface de l'hydrogel antimicrobien est supérieure à la concentration antimicrobienne dans un intérieur en vrac de l'hydrogel antimicrobien, et dans lequel d'environ 10 à environ 50 % de l'agent antimicrobien sont libérés de l'hydrogel sur une période inférieure à 24 heures.

2. Hydrogel antimicrobien selon la revendication 1, dans lequel après la libération d'environ 10 à environ 50 % de l'agent antimicrobien sur une période inférieure à 24 heures, la partie restante de l'agent antimicrobien est sensiblement conservée dans l'hydrogel.

3. Hydrogel antimicrobien selon la revendication 1, dans lequel l'hydrogel comprend des polymères de monomères de 4-acryloylmorpholine et de glycérol.

4. Hydrogel antimicrobien selon la revendication 1, dans lequel l'agent antimicrobien est sélectionné dans le groupe des sels de bis-biguanide (digluconate de chlorhexidine, diacétate de chlorhexidine, dichlorhydrate de chlorhexidine, diphosphanilate de chlorhexidine), de la rifampine, de la minocycline, des composés d'argent (chlorure d'argent, oxyde d'argent, sulfadiazine d'argent), du triclosan, du dichlorhydrate d'octénidine, de l'olanéxidine, de l'aléxidine, des composés d'ammonium quaternaires (chlorure de benzalkonium, chlorure de tridodécyl méthyl ammonium, chlorure de didécyl diméthyl ammonium, chlorure de chloroallyl hexaminium, chlorure de benzéthonium, chlorure de méthylbenzétonium, bromure de cétyl triméthyl ammonium, chlorure de cétyl pyridinium, chlorure de dioctyldiméthyl ammonium), des glycoprotéines séquestrant le fer (lactoferrine, ovotransferrine/conalbumine), des polypeptides cationiques (protamine, polylysine, lysozyme), des tensioactifs (SDS, Tween-80, surfactine, nonoxynol-9), de la zinc pyrithione, des antibiotiques à large spectre (quinolones, fluoroquinolones, aminoglycosides et sulfonamides), et agents antiseptiques (iode, méthénamine, nitrofurantoïne, acide nalidixique), et des combinaisons de ceux-ci.

5. Pansement pour plaie formé en utilisant l'hydrogel antimicrobien selon la revendication 1, dans lequel l'hydrogel antimicrobien ne gonfle pas significativement lorsque l'agent antimicrobien est libéré.

6. Pansement selon la revendication 5, dans lequel le pansement est sélectionné dans le groupe consistant en un pansement pour plaie, un pansement chirurgical, et un pansement pour site de cathéter.

7. Pansement selon la revendication 5 pour son utilisation dans l'accélération de la cicatrisation d'une plaie à laquelle ledit pansement pour plaie est appliqué.

8. Pansement pour plaie selon la revendication 5 pour son utilisation dans la prévention et/ou la réduction de l'incidence d'infection dans une paie à laquelle ledit pansement pour plaie est appliqué.
